(19) Europäisches Patentamt
European Patent Office
Office européen des brevets

(11) Numéro de publication: **0 366 544 A1**

# (12) DEMANDE DE BREVET EUROPEEN

(21) Numéro de dépôt: **89402943.8**

(51) Int. Cl.5: **C01B 25/37**

(22) Date de dépôt: **25.10.89**

Le titre de l'invention a été modifié (Directives relatives à l'examen pratiqué à l'OEB, A-III, 7.3)

(30) Priorité: **28.10.88 FR 8814122**

(43) Date de publication de la demande: **02.05.90 Bulletin 90/18**

(84) Etats contractants désignés: **AT BE CH DE ES FR GB GR IT LI LU NL SE**

(71) Demandeur: **BOEHRINGER INGELHEIM FRANCE**
**6, rue Léo Delibes**
**F-75116 Paris Cédex(FR)**

(72) Inventeur: **Cohen, Gilbert**
**37, avenue Charles Floquet**
**F-75007 Paris(FR)**
Inventeur: **Maury, Marc**
**16, rue des Glaieuls**
**F-24000 Périgueux(FR)**

(74) Mandataire: **Clisci, Serge et al**
**S.A. FEDIT-LORIOT CONSEILS EN PROPRIETE INDUSTRIELLE 38, avenue Hoche**
**F-75008 Paris(FR)**

## (54) Procédé de préparation d'un gel de phosphate de bismuth.

(57) La présente invention concerne un procédé de préparation d'un gel de phosphate de bismuth comprenant la réaction du sous-nitrate de bismuth avec l'acide phosphorique, et qui est caractérisé en ce que :

a) on dissout le sous-nitrate de bismuth dans de l'acide nitrique commercial ayant une densité de l'ordre de 1,41, et porte puis maintient la solution résultante à 40° C ;

b) on dilue l'acide phosphorique avec de l'eau purifiée jusqu'à ce que l'on obtienne une concentration en acide phosphorique comprise entre 20 et 122 g/l, puis porte et maintient à 40° C la température de la solution résultante ;

c) on introduit à 40° C sous agitation la solution diluée d'acide phosphorique dans la solution de sous-nitrate de bismuth ;

d) on maintient le milieu réactionnel à 40° C sous agitation douce pendant une durée supérieure ou égale à 0,5 h ;

e) on concentre le précipité formé pour obtenir un gelde consistance crémeuse que l'on rince avec de l'eau puis concentre à nouveau, jusqu'à ce que le gel ainsi rincé ait une acidité résiduelle inférieure à 0,1 N ; puis lave ledit gel au moyen d'une solution de bicarbonate de sodium à 15-25 g/l ; et lave enfin le précipité résultant.

EP 0 366 544 A1

# NOUVEAU PROCEDE DE PREPARATION D'UN GEL DE PHOSPHATE DE BISMUTH

## DOMAINE DE L'INVENTION

La présente invention concerne un nouveau procédé de préparation d'un gel de phosphate de bismuth.

## ART ANTERIEUR

On sait, notamment, des brevets français FR-B-2 043 490 et FR-B-2 153 173 que le phosphate colloïdal comprenant 70 % en poids de phosphate de bismuth et 30 % en poids d'eau, a déjà été préconisé en thérapeutique en tant que médicament pour le traitement des ulcères, des gastrites et des duodénites.

On sait aussi que ledit phosphate de bismuth colloïdal présente sous forme de gel a été signalé comme étant susceptible d'être éventuellement toxique ; par suite son exploitation en thérapeutique a été fortement limitée dans un grand nombre de pays. Il se trouve que l'éventuelle toxicité résulte de difficultés rencontrées au cours de la synthèse du phosphate de bismuth colloïdal lors de la reproduction des procédés de préparation connus.

Dans le cas d'ulcères de l'estomac et/ou du duodénum, on sait que l'élément actif est le bismuth et que la source de bismuth, que l'on administre par voie orale, est un sel de bismuth qui intervient en tant que véhicule ou support dudit bismuth. Ainsi, outre le gel de phosphate de bismuth, on sait que l'industrie pharmaceutique a commercialisé dans le passé d'autres sels en tant que source de bismuth, notamment les aluminate de bismuth, sous-carbonate de bismuth, sous-citrate de bismuth, citrate de bismuth, sous-gallate de bismuth, sous-nitrate de bismuth, tartrate de bismuth, sous-salicylate de bismuth et dicitratobismuthate de tripotassium (voir à cet effet la demande de brevet européen publiée EP-A-0 206 625).

On a aussi constaté la présence de bactéries telles que le Campylobacter pylori (également dénommé Campylobacter pyloridi ou Campylobacter pyrolidis) et les souches analogues dans les tissus ulcéreux [voir à cet effet les articles de B. MARSHALL et al., Digestion 37 suppl. 2, pages 16-30, (1987) et de C.S. GOODWIN et al., Journal of Antimicrobial Chemotherapy 17, pages 309-314, (1986) notamment].

On sait, par ailleurs, que des praticiens ont déjà prescrit dans le passé à leurs patients souffrant d'ulcères et/ou de gastrites un traitement comprenant l'administration par voie orale, d'une part, d'un sel de bismuth, et d'autre part, un agent anti-infectieux (notamment un antibiotique). Le succès d'un tel traitement est confirmé par EP-A-0 206 625. La solution technique préconisée par EP-A-0 206 625 d'un traitement associé sel de bismuth/anti-infectieux résulte de la constatation que le bismuth ainsi administré recouvre les zones de l'ulcère alors que l'anti-infectieux détruit ou inhibe le Campylobacter pylori et les bactéries analogues qui sont présentes dans les tissus ulcéreux.

Le procédé de préparation du gel de phosphate de bismuth colloïdal, qui est décrit dans FR-B-2 043 490, comprend la réaction à une température d'environ 40 +/- 2 °C (au moins en fin de réaction) d'une solution nitrique contenant environ 300 à 400 g/l de sous-nitrate de bismuth (ayant une teneur en $Bi_2O_3$ de 80 % en poids) avec une solution aqueuse contenant environ 90 à 160 g/l d'acide phosphorique à 85 % . Le rapport pondéral acide phosphorique /sous nitrate de bismuth recommandé suivant ledit brevet est d'environ 1/2.

Le brevet français FR-B-2 153 173 concerne l'obtention du phosphate neutre de bismuth colloïdal suivant un procédé consistant à mettre en présence en proportions équimoléculaires une solution aqueuse d'acide phosphorique contenant 125 g/l d'acide phosphorique et une solution de sous-nitrate de bismuth contenant 300 g/l de sous-nitrate de bismuth, à maintenir ce mélange sous agitation à 40-45 °C pendant 12 heures environ, à neutraliser la solution surnageante, et à laver le précipité à l'eau et au bicarbonate de sodium jusqu'à neutralité.

Le brevet FR-B-2 153 173 prévoit une durée de contact de 12 heures environ des ingrédients du milieu réactionnel constitués par la solution aqueuse de sous-nitrate de bismuth, d'une part, et la solution aqueuse d'acide phosphorique, d'autre part. Il se trouve que cette durée est arbitraire et ne procure aucun élément favorable en ce qui concerne la qualité du produit fini et/ou le rendement de la réaction.

Ledit brevet prévoit également un lavage du phosphate de bismuth après sédimentation sous la forme de particules d'une taille voisine du micromètre [cf page 1, lignes 37-39 de FR-B-2 153 173]. Or il se trouve que (i) la sédimentation est trop lente pour assurer une décantation efficace du phosphate de bismuth colloïdal et que (ii) le lavage ou rinçage dudit phosphate de bismuth tel que préconisé est insuffisant pour éliminer toutes les traces de phosphate, de nitrate et de carbonate solubles et des sels correspondants.

En effet quand on calcule la vitesse théorique de sédimentation de particules dans un milieu visqueux

selon la loi de Stocks suivant la relation

$$V = \frac{2r^2 (d_1-d_2)g}{9n}$$

où
V = vitesse de sédimentation (en cm/s),
r = rayon des particules (en cm),
$d_1$ = densité de la phase dispersée (en g/cm$^3$),
$d_2$ = densité de la phase dispersante (en g/cm$^3$),
g = accélération de la pesanteur (980 cm/s$^2$),
n = viscosité newtonienne de la phase dispersante [n($H_2O$) = 0,0101 poise (soit environ 1 cP ; 1 cP correspond approximativement à $10^{-3}$ Pa.s)],
on a, pour r = 1 micromètre (i.e. $10^{-4}$ cm),$d_1$ = 6 g/cm$^3$ ;
$d_2(H_2O)$ = 1 g/cm$^3$, et n = 1 cP, V = 0,001 cm/s environ, soit une distance parcourue en 1 heure par une particule de phosphate de bismuth voisine de 3,6 cm.

**BUT DE L'INVENTION**

Il existe un besoin en phosphate de bismuth colloïdal pour le traitement des ulcères, des gastrites et des duodénites. Dans cette optique, on se propose selon l'invention de fournir un nouveau procédé de synthèse du phosphate de bismuth colloïdal qui ne présente pas les inconvénients précités de l'art antérieur.

**OBJET DE L'INVENTION**

Suivant l'invention, on préconise un nouveau procédé de préparation d'un gel de phosphate de bismuth comprenant la réaction du sous-nitrate de bismuth avec l'acide phosphorique, qui est caractérisé en ce que

1°) on dissout le sous-nitrate de bismuth dans de l'acide nitrique commercial ayant une densité de l'ordre de 1,41, et porte puis maintient la solution résultante à 40°C ;

2°) on dilue l'acide phosphorique avec de l'eau purifiée (notamment choisie parmi l'ensemble comprenant l'eau distillée, l'eau bidistillée ou mieux l'eau déminéralisée ayant une résistivité supérieure ou égale à $10^5$ Ω.cm) jusqu'à ce que l'on obtienne une concentration en acide phosphorique comprise entre 20 et 122 g/l, puis porte et maintient à 40°C la température de la solution résultante ;

3°) on introduit à 40°C sous agitation la solution diluée d'acide phosphorique obtenue suivant le stade 2°) dans la solution de sous-nitrate de bismuth obtenue suivant le stade 1°) ;

4°) on maintient le milieu réactionnel obtenu suivant le stade 3°) à 40°C sous agitation douce pendant une durée supérieure ou égale à 0,5 h ;

5°) on concentre le précipité formé au cours du stade 4°) selon une technique choisie parmi l'ensemble comprenant les filtration et centrifugation, pour obtenir un gel de consistance crémeuse ;

6°) on rince le gel ainsi obtenu en le diluant avec de l'eau puis en le concentrant à nouveau suivant le stade 5°), jusqu'à ce que le gel ainsi rincé ait une acidité résiduelle inférieure à 0,1 N ;

7°) on lave ledit gel ainsi obtenu au stade 6°) d'une solution de bicarbonate de sodium à 15-25 g/l ;

8°) on lave le précipité résultant qui contient essentiellement le phosphate de bismuth sous forme de gel en association avec de l'eau, en procédant à nouveau à une opération de concentration suivant le stade 5°) et à une opération de rinçage et de concentration suivant le stade 6°).

Pour obtenir en produit conforme aux prescriptions des pharmacopées française et étrangères et éviter la présence d'impuretés dans le phosphate de bismuth final utilisable en thérapeutique, il est impératif de mettre en oeuvre les stades 1°) à 8°) du présent procédé.

La dissolution du sous-nitrate de bismuth dans l'acide nitrique conduit à la formation de nitrate de bismuth qui cristallise en moins d'une heure si la température de la solution résultante n'est pas maintenue

à une température supérieure ou égale à 40° C. De plus si le milieu réactionnel du stade 1°) n'est pas maintenu à une température voisine de 40° C on obtient un phosphate de bismuth impur contenant comme impureté principale du nitrate de bismuth. En conséquence, il faut opérer à 40° C, en portant et maintenant à cette température les solutions des stades 1°) et 2°), puis en effectuant les opérations des stades 3°) et 4°) à cette même température.

De façon pratique, les opérations de concentration [suivant le stade 5°)], de rinçage [suivant le stade 6°)] et de lavage [suivant les stades 7°) et 8°)] seront effectuées à une température comprise entre la température ambiante (15-25° C) et la température de 40° C, la température préférée étant de 40° C.

Il est important de respecter l'ordre d'introduction des réactifs du stade 3°). En effet, si l'on verse la solution nitrique de sous-nitrate de bismuth dans la solution d'acide phosphorique, on obtient un phosphate de bismuth, ayant une granulométrie très élevée, d'une part, et une vitesse de sédimentation trop élevée qui implique un risque d'entraînement de nitrate de bismuth dans le phosphate de bismuth formé, d'autre part. En fait suivant l'invention, on préconise des modalités opératoires qui donnent une sédimentation du phosphate de bismuth qui permette de limiter la durée de réaction du stade 4°) et convienne pour les futures opérations de rinçage et de lavage. En d'autres termes, on préconise suivant l'invention une technique réactionnelle qui procure une granulométrie particulière et par suite une sédimentation du phosphate de bismuth d'une vitesse suffisamment élevée se situant entre (i) la vitesse relativement lente (12 h de réaction quand on verse l'acide phosphorique dans la solution de sous-nitrate de bismuth) de FR-B-2 153 173, et (ii) la vitesse relativement grande (qui est néfaste pour la qualité du produit final) quand on verse la solution nitrique de sous-nitrate de bismuth dans l'acide phosphorique.

La qualité du phosphate de bismuth est en effet fonction de la concentration des composants intervenant au stade 3°) et par suite au stade 4°). Cette concentration influence la répartition granulométrique du phosphate de bismuth colloïdal obtenu : plus la concentration du milieu réactionnel est élevée, plus le précipité obtenu au stade 3°) est dense. Aussi selon une des caractéristiques de l'invention on préconise, pour la mise en oeuvre du stade 3°), de préparer :

(A) au stade 1°), une solution renfermant 200 à 450 g/l (et mieux 250 à 400 g/l) de sous-nitrate de bismuth dans de l'acide nitrique commercial [solution aqueuse contenant en poids 68 %

$$(d_4^{20°C}= 1,410) \text{ à } 70 \% \ (d_4^{20°C}= 1,413) \text{ de } HNO_3],$$

et

(B) au stade 2°), une solution aqueuse renfermant 20 à 122 g/l d'acide phosphorique. de façon que le rapport pondéral R acide phosphorique / sous-nitrate de bismuth, qui est utilisé au stade 3°), soit compris entre 1/2,5 et 1/3, et mieux entre 1/2,6 et 1/2,8.

On remarquera à ce sujet que, selon FR-B-2 043 490, la concentration du sous-nitrate de bismuth est de 300 à 400 g/l, celle de l'acide phosphorique est de 76,5 (i.e. : 90 x 0,85) à 136 (i.e. : 160 x 0,85)g/l et le rapport pondéral R préféré est de l'ordre de 1/2 (sic), et que, selon FR-B-2 153 173, la concentration du sous-nitrate de bismuth est de 300 g/l, celle de l'acide phosphorique est de 125 g/l et le rapport pondéral R est de 1/2,4.

La matière première utilisée au stade 1°) est de préférence un sous-nitrate de bismuth dit lourd ayant une teneur en $B_i$ de 70 à 74 % en poids (soit approximativement une teneur en $Bi_2O_3$ de 79 à 82 % en poids). Le sous-nitrate de bismuth dit léger est également utilisable comme matière première.

La dilution du stade 2°) est réalisée avec une eau purifiée comme indiqué ci-dessus. De préférence on utilisera une eau déminéralisée ayant une résistivité supérieure ou égale à $10^5$ Ω.cm, et mieux supérieure ou égale à $10^6$ Ω.cm. De façon pratique, l'acide phosphorique utilisé comme matière première devant être diluée, sera un produit du commerce, notamment une solution aqueuse conmerciale contenant 85 % en poids de $H_3PO_4$ (solution commerciale ayant une densité $d^{25°C} = 1,6850$).

Selon l'invention, on préparera avantageusement une solution d'acide phosphorique renfermant une quantité de $H_3PO_4$ supérieure à 20 g/l et inférieure ou égale à environ 120 g/l, le rapport pondéral R utilisé au stade 3°) étant compris entre 1/2,5 et 1/3 et mieux entre 1/2,6 et 1/2,8.

Les eaux de rinçage et de lavage des stades 6°) et 8°) seront avantageusement des eaux purifiées comme indiqué ci-dessus (eau distillée, eau bidistillée ou eau déminéralisée) ; la solution de bicarbonate de sodium du stade 7°) sera avantageusement préparée par dissolution dudit bicarbonate de sodium dans de l'eau purifiée.

Suivant une autre caractéristique de l'invention, on prévoit, à l'issue du stade 8°), l'opération complémentaire suivante :

9°) on sèche le gel ainsi lavé et obtenu suivant le stade 8°), jusqu'à ce que l'on obtienne un gel de phosphate de bismuth séché ayant une teneur résiduelle en eau inférieure ou égale à 4 % en poids.

Le stade 9°) est facultatif quand on réalise le séchage on opère dans une étuve ventilée. La température de séchage peut être comprise entre 40°C et 100°C, voire supérieure à 100°C. Ce séchage est une simple déshydratation d'un produit minéral sans risque de dégradation de celui-ci.

Enfin, la concentration du stade 5°) comprend une centrifugation dite simple; une centrifugation dite tangentielle, une centrifugation ou une ultracentrifugation.

Selon le meilleur mode de mise en oeuvre du procédé de l'invention, on préconise de mettre en oeuvre les modalités des stades 3°) et 4°) pendant une durée de réaction de 0,5 à 3 h, et, d'entreprendre au moins trois rinçages successifs du produit concentré obtenu suivant le stade 5°). En pratique on reproduira à cet effet au moins 3 fois de suite les modalités du stade 6°), avant de mettre en oeuvre le stade 7°), le stade 9°) sus-visé étant ici réalisé après le stade 8°).

D'autres avantages et caractéristiques de l'invention seront mieux compris à la lecture qui va suivre d'exemples de préparation et de résultats d'essais comparatifs. Bien entendu, l'ensemble de ces éléments n'est pas limitatif mais est donné à titre d'illustration.

## EXEMPLE 1

On opère selon les modalités opératoires du meilleur mode de réalisation précité :

stade 1°) : on dissout 40 g de sous-nitrate de bismuth lourd ayant une teneur en $Bi_2O_3$ de 80 % en poids dans 150 ml d'une solution aqueuse d'acide nitrique renfermant 69 % en poids de $HNO_3$, on porte puis maintient la solution résultante à 40°C ;

stade 2°) : on dilue 17 g d'acide phosphorique ayant une teneur en $H_3PO_4$ de 85 % en poids dans 200 ml d'eau déminéralisée ayant une résistivité supérieure ou égale à $10^5$ Ω.cm et mieux supérieure ou égale à $10^6$ Ω.cm, jusqu'à ce que l'on ait une solution aqueuse renfermant de 20 à 122 g/l de $H_3PO_4$ ; on porte puis maintient à 40°C la température de la solution résultante ;

stade 3°) : on introduit à 40°C sous agitation la solution diluée d'acide phosphorique obtenue suivant le stade 2°) dans la solution de sous-nitrate de bismuth obtenue suivant le stade 1°), le rapport pondéral R tel que défini ci-dessus étant de 1/2,76 ;

stade 4°) : on maintient le milieu réactionnel obtenu suivant le stade 3°) à 40°C sous agitation douce pendant une durée de 0,5 h ;

stade 5°) : on concentre le précipité formé au cours du stade 4°) selon une technique choisie parmi l'ensemble comprenant les filtration et centrifugation, pour obtenir un gel de consistance crémeuse ;

stade 6°) : on rince trois fois le gel ainsi obtenu en le diluant avec de l'eau, chaque rinçage étant suivi d'une concentration suivant le stade 5°) ci-dessus, jusqu'à ce que le gel ainsi rincé ait une acidité résiduelle inférieure à 0,1N ;

stade 7°) : on lave ledit gel ainsi obtenu au stade 6°) au moyen d'une solution de bicarbonate de sodium à 15-25 g/l ;

stade 8°) : on lave le précipité résultant qui contient essentiellement le phosphate de bismuth sous forme de gel en association avec de l'eau, en procédant à nouveau à une opération de concentration suivant le stade 5°) et à une opération de rinçage et de concentration suivant le stade 6°) ;

stade 9°) : on sèche le gel ainsi lavé suivant le stade 8°), à une température de 40-100°C ou supérieure à 100°C, jusqu'à ce que l'on obtienne un gel de phosphate de bismuth séché ayant une teneur résiduelle en eau inférieure ou égale à 4 % en poids.

## EXEMPLES 2-6

On prépare un gel de phosphate de bismuth selon le procédé de l'exemple 1 en tenant compte des modalités consignées dans le tableau I ci-après. Le produit de l'exemple 6 est préparé avec une durée de réaction de 12 h identique à celle préconisée par FR-A-2 153 173.

## EXEMPLES COMPARATIFS CP1 ET CP2

On prépare des gels selon le procédé décrit objet du brevet FR-B-2 153 173, le premier (CP1) avec un

rapport R de 2,4, et le second (CP2) avec un rapport R de 3,4. Les modalités opératoires correspondantes sont également consignées dans le tableau I ci-après.

TABLEAU I

| Produit | durée | Composants | | | | R |
|---|---|---|---|---|---|---|
| | (a) | (b) | (c) | (d) | (e) | (f) |
| Ex 1 | 0,5 h | 40 g (267 g/l) | 150 ml | 17 g (72,2 g/l) | 200 ml | 1/2,76 |
| Ex 2 | 0,5 h | 40 g (267 g/l) | 150 ml | 17 g (26,1 g/l) | 400 ml | 1/2,76 |
| Ex 3 | 1,5 h | 40 g (267 g/l) | 150 ml | 17 g (24,0 g/l) | 600 ml | 1/2,76 |
| Ex 4 | 3 h | 40 g (400 g/l) | 100 ml | 17 g (20,6 g/l) | 700 ml | 1/2,76 |
| Ex 5 | 0,5 h | 40 g (308 g/l) | 130 ml | 17 g (120,4 g/l) | 120 ml | 1/2,76 |
| Ex 6 | 12 h | 40 g (350 g/l) | 114 ml | 17 g (77,3 g/l) | 187 ml | 1/2,76 |
| CP1 | 12 h | 40 g (350 g/l) | 114 ml | 22 g (100 g/l) | 187 ml | 1/2,14 |
| CP2 | 12 h | 40 g (350 g/l) | 114 ml | 15 g (63,2 g/l) | 187 ml | 1/3,14 |
| Notes : | | | | | | |

(a) durée de réaction ;
(b) quantité de sous-nitrate de bismuth contenant 80 % en poids de $Bi_2O_3$, avec entre parenthèses la concentration en pourcentage en poids dudit sous-nitrate dans la solution nitrique ;
(c) volume de la solution d'acide nitrique contenant 69 % en poids de $HNO_3$ utilisée pour dissoudre le sous nitrate de bismuth ;
(d) quantité d'acide phosphorique contenant 85 % en poids de $H_3PO_4$, avec entre parenthèses la concentration en pourcentage en poids de $H_3PO_4$ dans la solution diluée d'acide pho phorique;
(e) volume d'eau déminéralisée (résistivité supérieure à $10^6\,\Omega$.cm) ;
(f) rapport pondéral $H_3PO_4$/sous-nitrate de bismuth au début de la réaction.

## ANALYSE

On a consigné dans le tableau II ci-après les rendements obtenus pour les exemples 1-6 et les exemples comparatifs CP1 et CP2 lors de la synthèse des gels de phosphate de bismuth ainsi que les dosages des teneurs (exprimées en pourcentage en poids) en bismuth, ions $PO_4^{3-}$ et eau desdits gels.

Les résultats obtenus mettent en évidence l'intérêt de l'invention en ce qui concerne la qualité du gel de phosphate de bismuth et le rendement de la synthèse, d'une part, et surtout les économies d'énergie qui résultent de la diminution de la durée de réaction de 12 h à 0,5-3 h, d'autre part.

TABLEAU II

| Produit | Bi | $PO_4^{3-}$ | $H_2O$ | Rendement |
|---|---|---|---|---|
| Ex 1 | 66,4 % | 29,3 % | 1,7 % | 36 g |
| Ex 2 | 67,8 % | 30 % | 4 % | 38 g |
| Ex 3 | 67,7 % | 29,6 % | 4 % | 37 g |
| Ex 4 | | | | 40 g |
| Ex 5 | 67,7 % | | | 31 g |
| Ex 6 | 66,1 % | 30,2 % | 4 % | 30 g |
| CP 1 | 66,2 % | | 4,1 % | 26 g |
| CP 2 | 66,3 % | | 4,5 % | 24 g |

**Revendications**

1. Procédé de préparation d'un gel de phosphate de bismuth comprenant la réaction du sous-nitrate de bismuth avec l'acide phosphorique, et qui est caractérisé en ce que :

1°) on dissout le sous-nitrate de bismuth dans de l'acide nitrique commercial ayant une densité de l'ordre de 1,41, et porte puis maintient la solution résultante à 40°C ;

2°) on dilue l'acide phosphorique avec de l'eau purifiée (notamment choisie parmi l'ensemble comprenant l'eau distillée, l'eau bidistillée ou mieux l'eau déminéralisée ayant une résistivité supérieure ou égale à $10^5 \Omega$.cm) jusqu'à ce que l'on obtienne une concentration en acide phosphorique comprise entre 20 et 122 g/l, puis porte et maintient à 40°C la température de la solution résultante ;

3°) on introduit à 40°C sous agitation la solution diluée d'acide phosphorique obtenue suivant le stade 2°) dans la solution de sous-nitrate de bismuth obtenue suivant le stade 1°) ;

4°) on maintient le milieu réactionnel obtenu suivant le stade 3°) à 40°C sous agitation douce pendant une durée supérieure ou égale à 0,5 h ;

5°) on concentre le précipité formé au cours du stade 4°) selon une technique choisie parmi l'ensemble comprenant les filtration et centrifugation, pour obtenir un gel de consistance crémeuse ;

6°) on rince le gel ainsi obtenu en le diluant avec de l'eau puis en le concentrant à nouveau suivant le stade 5°), jusqu'à ce que le gel ainsi rincé ait une acidité résiduelle inférieure à 0,1 N ;

7°) on lave ledit gel ainsi obtenu au stade 6°) au moyen d'une solution de bicarbonate de sodium à 15-25 g/l ;

8°) on lave le précipité résultant qui contient essentiellement le phosphate de bismuth sous forme de gel en association avec de l'eau, en procédant à nouveau à une opération de concentration suivant le stade 5°) et à une opération de rinçage et de concentration suivant le stade 6°).

2. Procédé suivant la revendication 1, caractérisé en ce que :

au stade 1°) :

on prépare une solution renfermant 200 à 450 g/l de sous-nitrate de bismuth dans de l'acide nitrique commercial contenant 68-70 % en poids de $HNO_3$,

au stade 2°) :

on prépare une solution aqueuse d'acide phosphorique renfermant 20 à 122 g/l de $H_3PO_4$, et

au stade 3°) :

on verse à 40°C sous agitation la solution diluée d'acide phosphorique dans la solution d'acide nitrique contenant le sous-nitrate de bismuth de telle façon que le rapport pondéral (R) $H_3PO_4$/sous-nitrate de bismuth soit compris entre 1/2,5 et 1/3.

3. Procédé suivant la revendication 1, caractérisé en ce que :

au stade 1°) :

on prépare une solution renfermant 250 à 400 g/l de sous-nitrate de bismuth dans de l'acide nitrique commercial contenant 68-70 % en poids de $HNO_3$,

au stade 2°) :

on prépare une solution aqueuse d'acide phosphorique renfermant 20 à 120 g/l de $H_3PO_4$, et

au stade 3°) :

on verse à 40°C sous agitation la solution diluée d'acide phosphorique dans la solution d'acide nitrique contenant le sous-nitrate de bismuth de telle façon que le rapport pondéral (R) $H_3PO_4$/sous-nitrate de bismuth soit compris entre 1/2,6 et 1/2,8.

4. Procédé suivant la revendication 1, caractérisé en ce que, au stade 4°), la durée de réaction est comprise entre 0,5 h et 3 h.

5. Procédé suivant la revendication 1, caractérisé en ce que le stade 6°) comprend au moins trois rinçages successifs du produit concentré obtenu suivant le stade 5°), chaque rinçage étant suivi d'une opération de concentration selon le stade 5°).

6. Procédé suivant la revendication 1, caractérisé en ce que le stade 8°) est suivi d'une opération complémentaire selon laquelle :

9°) on sèche le gel ainsi obtenu suivant le stade 8°), jusqu'à ce que l'on obtienne un gel de phosphate de bismuth séché ayant une teneur résiduelle en eau inférieure ou égale à 4 % en poids.

7. Procédé suivant la revendication 1, caractérisé en ce que les opérations des stades 5°) à 8°) sont effectués à une température comprise entre la température ambiante et la température de 40°C, et mieux à la température de 40°C.

8. Procédé suivant la revendication 1, caractérisé en ce que l'eau de rinçage ou lavage des stades 6°) et 8°) est une eau purifiée.

9. Procédé suivant la revendication 1, caractérisé en ce que la solution de bicarbonate de sodium

utilisée au stade 7°) est obtenue par dissolution de bicarbonate de sodium dans de l'eau purifiée.

Office européen des brevets

# RAPPORT DE RECHERCHE EUROPEENNE

Numero de la demande

EP 89 40 2943

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | FR-A-2 043 490 (J.P. RAUDNITZ)<br>--- | | C 01 B 25/37 |
| D,A | FR-A-2 153 173 (LABORATOIRES BIOTHERAX)<br>----- | | |
| | | | DOMAINES TECHNIQUES RECHERCHES (Int. Cl.5) |
| | | | C 01 B 25/00 |

Le présent rapport a été établi pour toutes les revendications

| Lieu de la recherche | Date d'achèvement de la recherche | Examinateur |
|---|---|---|
| LA HAYE | 30-11-1989 | BREBION J.CH. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant